# EUROPEAN PATENT SPECIFICATION

(11) **EP 1 664 718 B1**
(45) Date of publication and mention of the grant of the patent: **09.12.2009**
(21) Application number: 04783123.5
(22) Date of filing: 03.09.2004
(51) Int. Cl.: C12Q 1/68, G01N 33/574

(54) **DIAGNOSIS AND MONITORING OF HEPATOCELLULAR CARCINOMA**
DIAGNOSE UND ÜBERWACHUNG VON HEPATOZELLULÄREM KARZINOM
DIAGNOSTIC ET SURVEILLANCE DU CARCINOME HEPATOCELLULAIRE

(30) Priority: 05.09.2003 US 500657 P
(43) Date of publication of application: 07.06.2006
(73) Proprietor: Thomas Jefferson University, Philadelphia, PA 19107 (US); Saint Louis University, St. Louis MO 63114 (US); The United States of America as represented by the Department of Veterans Affairs, Washington, DC 20420 (US)
(72) Inventor: ROMANO, Patrick, R., Doylestown, PA 18901 (US); BLOCK, Timothy, M., Doylestown, PA 18901 (US); FIMMEL, Claus, J., Clayton, MO 63105 (US); NIKOLAEVA, Olga, Pipersville, PA 18947 (US)
(74) Representative: Ouzman, Beverley Nicola Claire
(86) International application number: PCT/US2004/028766
(87) International publication number: WO 2005/026687

(56) References cited:
- WO-A-03/010336
- KLADNEY RALEIGH D ET AL: "Expression of GP73, a resident golgi membrane protein, in viral and nonviral liver disease" HEPATOLOGY, vol. 35, no. 6, June 2002 (2002-06), pages 1431-1440, XP002445869 ISSN: 0270-9139
- ROMANO PATRICK R ET AL: "GP73, a resident Golgi membrane protein, appears in sera of patients with viral liver disease and hepatocellular cancer." HEPATOLOGY, vol. 38, no. 4 Suppl. 1, October 2003 (2003-10), page 768A, XP004624472 & 54TH ANNUAL MEETING OF THE AMERICAN ASSOCIATION FOR THE STUDY OF LIVER DISEASES; BOSTON, MA, USA; OCTOBER 24-28, 2003 ISSN: 0270-9139
- BLOCK T.M. ET AL: 'Use of targeted glycoproteomics to identify serum glycoproteins that correlate with liver cancer in woodchucks and humans.' PNAS. vol. 102, no. 3, January 2005, pages 779 - 784, XP002992310
- IFTIKHAR R. ET AL: 'Disease-and Cell-specific expression of GP73 in human Liver disease.' AMERICAN JOURNAL OF GASTROENTEROLOGY. vol. 99, no. 6, June 2004, pages 1087 - 1095, XP002992311

## Description

### Field of the Invention

The invention relates to the diagnosis of hepatocellular carcinoma (HCC) through detecting biological markers of HCC in the serum, in particular through the detection of GP73 protein in the serum. The invention also relates to the monitoring of subjects for the development of HCC, through evaluation of GP73 levels in the serum.

### Background of the Invention

HCC is the fifth-most prevalent cancer in the word. However, because the disease is often refractory to treatment, HCC is the third leading cause of worldwide cancer mortality, with a five-year survival rate following diagnosis of less than five percent.

The major etiology of HCC is chronic infection with either hepatitis B virus (HBV) or hepatitis C virus (HCV). The long latency period between HBV or HCV infection and HCC onset in this high-risk population provides an opportunity for early detection well before the onset of serious disease. Populations at risk for HCC that can therefore be monitored for biomarkers of disease as they become available. Moreover, as more therapeutic options become available, early detection of HCC is important to improving patient prognosis.

Currently, HCC disease status is typically monitored by physical assessment, ultrasound imaging of the liver or analysis of serum for a panel of markers. Since there is a good correlation between elevated levels of alpha fetoprotein (AFP) and the occurrence of HCC; determination of AFP levels is often included as a serum marker of disease. However, AFP as a sole indicator of HCC is of limited value, as this protein is often elevated in the absence of serious disease. Its value in the detection of HCV-associated HCC is even less clear. Nevertheless, even the limited correlation between AFP and HCC underscores the potential of serum as a source of biomarkers of liver disease.

The clinical disposition of HBV and HCV carriers can generally be divided into four clinical categories: inactive, active, cirrhotic and HCC. Although a given category is not necessarily a precursor to the succeeding, HCC may be considered an end stage to the progression of liver disease in an infected subject. To assist in early detection and disease prognosis, the identification of serum molecular markers (polypeptides, glycolipids and proteoglycans), whose abundance correlates with these clinical categories, would be highly desirable.

GP73 is a type II Golgi transmembrane protein that is expressed at high level in the hepatocytes of patients with viral hepatitis (Kladney, et al., 2000, Gene 249, 53-65). GP73 is constitutively expressed in biliary epithelial cells, and minimally expressed in normal hepatocytes. In contrast, livers of patients with giant-cell hepatitis display strong immunoreactivity to GP73 in multinucleated hepatocytes. GP73 mRNA and protein are expressed in highly differentiated HepG2 hepatoma cells after infection with viruses, including adenoviruses. Because GP73 is a Golgi transmembrane protein, it is not expected to exist in significant amounts in the serum, even in subjects with damaged or diseased livers.

Significant increases in whole-organ levels of GP73 have been found in liver disease due to viral causes (HBV, HCV) or nonviral causes (alcohol-induced liver disease, autoimmune hepatitis); see Kladney, et al., 2002, Hepatology 35(6):1431-40. Hepatocyte expression of GP73 is unregulated in diseased livers, regardless of etiology, whereas biliary epithelial cell expression does not change appreciably.

While these reports are interesting, detection of GP73 in liver cells requires the patient to submit to a liver biopsy, which is painful and inconvenient.

What is needed, therefore, is a simple and quick assay for determining the extent of liver disease and damage caused by hepatitis virus infection. A serum assay for a biomarker of HCC would provide that speed and simplicity.

WO 03/010336 discloses diagnostic markers for HCC, including a 63kD Golgi transmembrane protein deposited under accession No. NM-001518.1.

### Summary of the Invention

The presence of elevated levels of GP73 or a polypeptide of 25kDa +/- 5kDa which cross-reacts with a GP73-specific antibody, in the serum of a subject indicates whether the subject is suffering from or has developed HCC. Non-cancerous or pre-cancerous liver disorders do not appear to cause elevated serum levels of GP73.

The invention thus provides a method of diagnosing HCC in a subject, comprising determining the serum levels of GP73, a polypeptide of 25kDa +/- 5kDa which cross-reacts with a GP73-specific antibody, or both in a subject relative to serum levels of GP73 or the polypeptide in a control. Elevated levels of serum GP73, the polypeptide, or both in a subject indicates that the subject has HCC.

The invention also provides a method of monitoring a subject at risk for developing HCC, comprising determining the serum levels of GP73, a polypeptide of 25kDa +/- 5kDa which cross-reacts with a GP73-specific antibody, or both in the subject for at least two time points. An increase in serum GP73, said polypeptide or both in the subject relative to earlier time points indicates that the subject has developed HCC.

### Abbreviations

The following abbreviations are used herein:
AFP: alpha fetoprotein
ALT: alanine aminotransferase
ANOVA: analysis of variance
AST: asparagine transaminase
AUROC: area under a ROC curve
ECL: enhanced chemiluminescence
ELISA: enzyme-linked immunosorbent assay
GST: glutathione-S-transferase
HBV: hepatitis B virus
HBsAg: HBV surface antigen
HCC: hepatocellular carcinoma
HCV: hepatitis C virus
HIV: human immunodeficiency virus
MELD: model for end stage liver disease score
RIA: radio immunoassay
ROC: receiver operating characteristic
SDS-PAGE: sodium dodecyl sulfate polyacrylamide gel electrophoresis

### Brief Description of the Figures

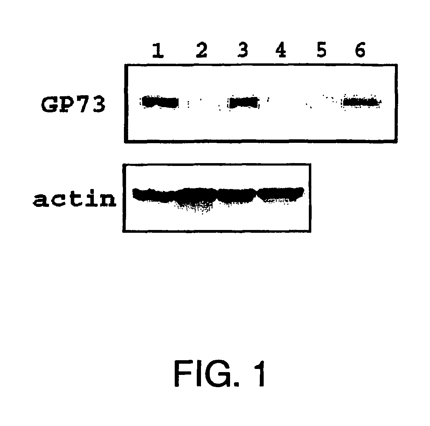

Figure 1 is an immunoblot analysis of GP73 in patients infected with HCV. The upper panel, marked "GP73," shows an immunoblot analysis with anti-GP73 rabbit polyclonal antibody against the following: HCC tissue lysates (lanes 1 and 2; 25 µg/lane); cirrhotic tissue lysate (lanes 3 and 4; 25 µg/lane); and sera (1 µl/ane) from the two HCV-infected patients with HCC (lanes 5 and 6). The lower panel, marked "actin," represents the same blot shown in the upper panel, after being stripped and then reprobed for actin as a control for protein loading.

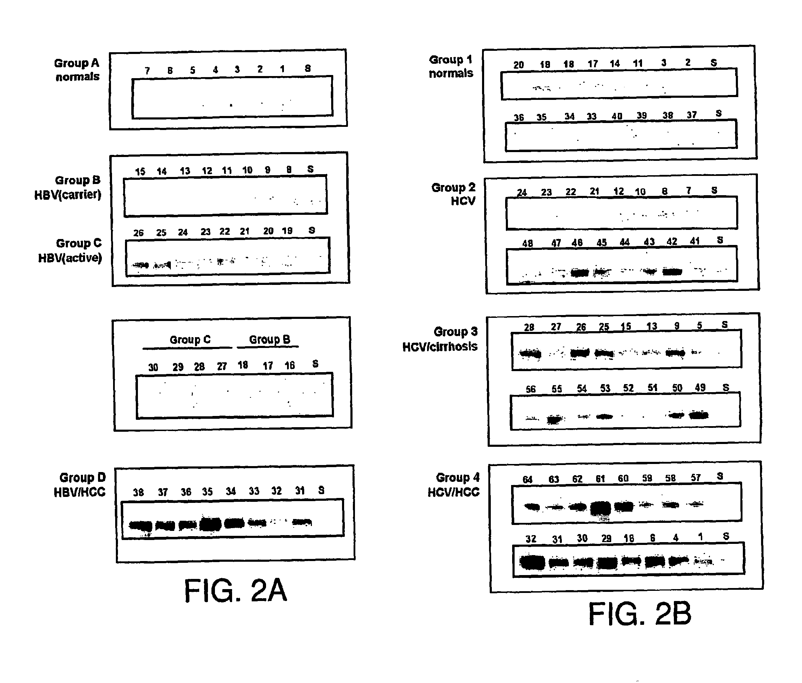

Figures 2A and 2B show the results of an immunoblot analysis with anti-GP73 specific antibody against human serum (0.5 µl) obtained from human subjects as follows: HBV-negative (Group A); HBV carrier with inactive infection (Group B); HBV carrier with active infection (Group C); HBV-associated HCC (Group D); HCV-negative (Group 1); chronic HCV infection (Group 2); HCV-related cirrhosis (Group 3); and HCV-related HCC (Group 4). A number assigned to each study subject for identification purposes is indicated above each lane. Commercially obtained, pooled sera from HCV-, HIV, and HBV-negative subjects (S) were used for normal controls.

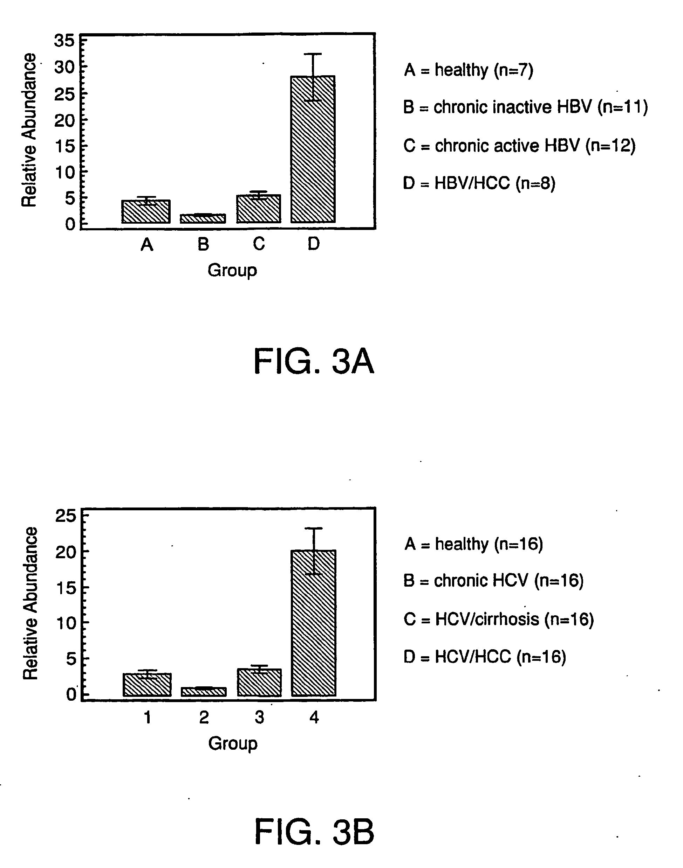

Figures 3A and 3B show the results of densitometric analyses of the immunoblot images of Figures 2A and 2B.

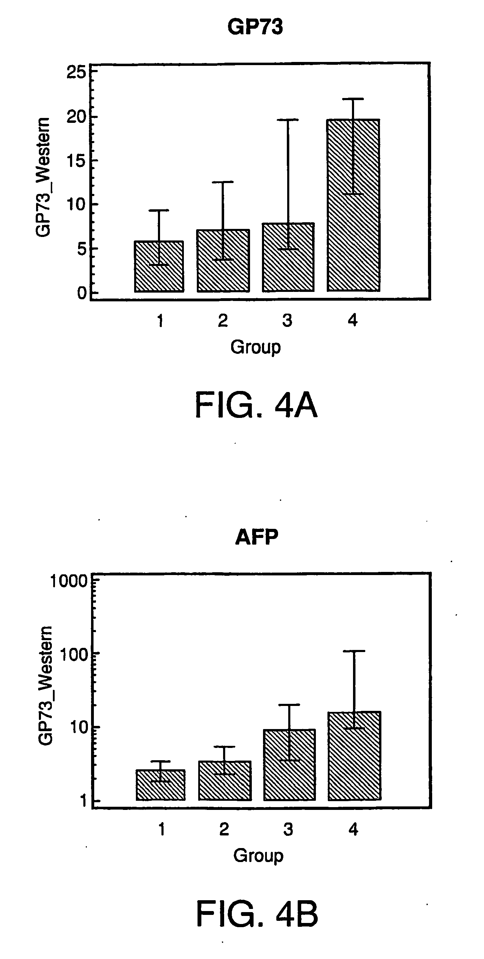

Figures 4A and 4B show the results of a study measuring GP73 (Fig. 4A) serum levels and α-fetoprotein (AFP) (Fig. 4B) serum levels in patients with HCV-associated liver disease and in control patients. The indicated GP73 levels are normalized to GP73 levels measured in control serum (Sigma). AFP levels are reported as ng/ml of serum. Error bars represent standard error of the mean.

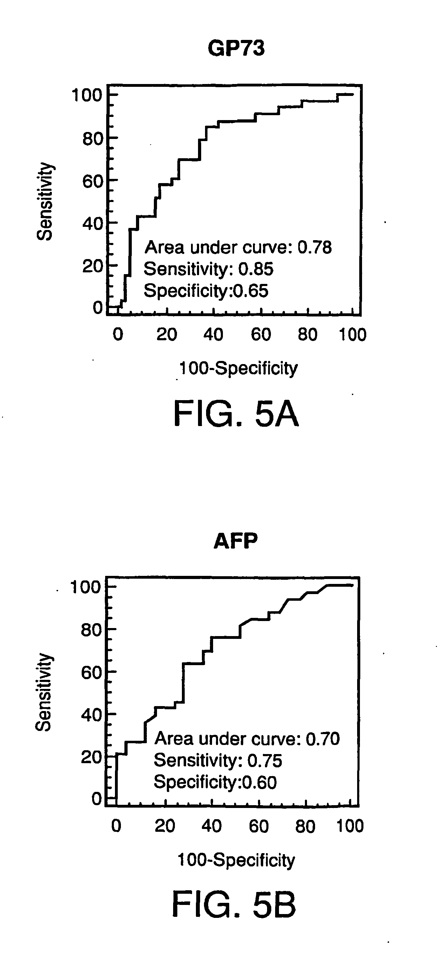

Figures 5A and 5B represent Receiver Operating Characteristic (ROC) curves generated based on the data of Figures 4A and 4B. The area under the ROC (AUROC) curves for GP73 and AFP are indicated in the inserts.

### Detailed Description of the Invention

GP73 is a 400 amino acid type II Golgi membrane protein of unknown function, that has an apparent molecular weight of approximately 73kDa. Kladney et al., 2000, Gene 249, 53-65. The nucleotide and deduced amino acid sequence of GP73 is disclosed in Kladney et al., 2000, supra, and in GenBank record Accession No. AF236056. The full-length GP73 cDNA, which comprises 3042 base pairs and contains a single open reading frame of 1200 base pairs, is represented herein as SEQ ID NO: 1. The amino acid sequence of GP73 is represented herein as SEQ ID NO: 2.

It has now been found that appreciable amounts of GP73 and a polypeptide having a molecular weight of approximately 25 kDa + 5 kDa, which cross reacts with a GP73-specific antibody, exist in the sera of individuals with HCC. The skilled artisan would reasonably expect that the polypeptide having a molecular weight of approximately 25 kDa + 5 kDa, which cross reacts with a GP73-specific antibody, is a fragment of GP73. Individuals with no liver disorders or infections of the liver, or individuals with non-cancerous or pre-cancerous liver disorders, have little or no GP73 or the abovementioned polypeptide in their sera. The appearance of GP73 or the polypeptide of 25kDa +/- 5kDa which cross-reacts with a GP73-specific antibody, in the sera of individuals previously diagnosed with a non-cancerous or pre-cancerous liver disorder indicates that the liver disorder has progressed to the cancerous state. Based on the initial characterization of GP73 as a resident Golgi transmembrane protein, it was not expected that GP73 would be found in the circulation, even in subjects with diseased or damaged livers. Throughout the instant disclosure, any reference to GP73, as a detectable molecular entity, is meant to include the full length GP73 protein and the polypeptide of 25kDa +/- 5kDa which cross-reacts with a GP73-specific antibody.

The invention thus provides a method of diagnosing HCC in a subject suspected of having HCC. The diagnostic method comprises determining the level of GP73 or a polypeptide of 25kDa +/- 5kDa which cross-reacts with a GP73-specific antibody in a serum sample obtained from the subject, and comparing the GP73 or polypeptide levels in that serum sample to the serum GP73 or polypeptide levels in a control. An elevated level of GP73 or polypeptide in the serum sample from the subject as compared to the control indicates that the subject has HCC.

An "elevated level" of GP73 in a serum sample from a subject as compared to a control means that the amount of GP73 protein per unit volume or unit mass is greater in the subject's serum sample than in the control. The level of GP73 can be expressed in absolute units of mass of protein per unit volume or unit mass; *e.g*., as picograms per microliter. The level of GP73 can also be expressed in arbitrary units, such as fluorescence or densitometric units, as determined relative to a control sample. In the Examples below, GP73 levels are expressed in arbitrary densitometric units.

While any elevated level of GP73 may be predictive of disease in the practice of this invention, preferably, the level of GP73 protein in a subject's serum sample is at least 2-fold, for example at least 3-fold, at least 4-fold, at least 5-fold, at least 6-fold, at least 7-fold, or at least 8-fold greater than the GP73 level in the control.

As used herein, a "subject" is any animal suspected of having HCC. Preferably, the subject is a mammal; for example an ovine, bovine, porcine, equine, canine, feline, rodent or primate. More preferably, the subject is a rodent, for example a mouse or rat. Even more preferably, the subject is a primate, for example a human. In particularly preferred embodiments, the subject is a human.

As used herein, a "serum sample" is any biological sample comprising serum. It is understood that a serum sample for use in the present methods can contain other components, in particular blood components. Thus, whole blood samples, or blood samples which have been only partially fractionated or separated but which still contain serum, are considered "serum samples" for purposes of the present invention. One skilled in the art can readily obtain serum samples, for example by using conventional blood drawing techniques. Furthermore, the presence of preservative, anticoagulants or other chemicals in the serum sample should not inhibit the detection of GP73. As used herein, "serum samples" also include controls or control samples.

As used herein, a "control" or "control sample" refers to one or more serum samples taken from at least one individual who has tested negative for any hepatitis infection, or who does not have HCC or any other liver disorder. Preferably, the control or control serum sample is obtained from at least one individual who has tested negative for any hepatitis infection, and who does not have HCC or any other liver disorder. In particularly preferred embodiments, the control comprises serum samples obtained from a population of individuals who have tested negative for any hepatitis infection and do not have HCC or any other liver disorder. It is understood that when the control comprises multiple serum samples, the serum GP73 level can be expressed as the arithmetic mean, median, mode or other suitable statistical measure of the GP73 level measured in each serum sample. Multiple control serum samples can also be pooled, and the GP73 level of the pooled samples can be determined and compared to the subject's serum sample.

One skilled in the art can readily obtain control serum samples, for example by conventional blood drawing techniques or by obtaining commercial serum samples which are from individuals who do not have HCC or any liver disorder. Commercial serum samples suitable for use as controls in the present methods can be obtained, for example, from Sigma Chemical Co., St. Louis, MO.

Any technique suitable for detecting serum GP73 levels can be used with the present methods, such as for example a GP73-specific biomolecular interaction, which includes but is not limited to antibody-based assays, aptamer-based assays, receptor and ligand assays; enzyme activity assays, and allosteric regulator binding assays. Techniques for detecting protein concentrations in a serum sample are within the skill in the art. Preferably, serum GP73 levels are detected by immunoassays such as Western blot analysis, radioimmunoassay (RIA), immunofluorescent assay, chemiluminescent assay, or enzyme-linked immunosorbent assay (ELISA). Immunoassays suitable for use in the present methods are described, for example, U.S. Pat. Nos. 5,976,809; 5,965,379; 5,571,680; 5,279,956; and 6,579,684 .

Detection of serum GP73 levels by Western blot analysis is described, for example, in Kladney et al., 2002, Hepatology 35: 1431-1440 , and in the Examples below.

The antibody used to detect GP73 levels in serum samples can comprise a polyclonal or monoclonal antibody. The antibody can comprise an intact antibody, or antibody fragments capable of specifically binding GP73 protein. Such fragments include, but are not limited to, Fab and F(ab')₂ fragments. Thus, as used herein, the term "antibody" includes both polyclonal and monoclonal antibodies. The term "antibody" means not only intact antibody molecules, but also includes fragments thereof which retain antigen binding ability.

Appropriate polyclonal antibodies can be prepared by immunizing appropriate host animals with GP73 protein and collecting and purifying the antisera according to conventional techniques known to those skilled in the art. Preparation of polyclonal anti-GP73 antibodies is described, for example, in Kladney et al., 2000, *supra.*

Monoclonal antibodies can be prepared by following the classical technique of Kohler and Milstein, Nature 254:493-497 (1975), as further elaborated in later works such as Monoclonal Antibodies, - Hybridomas: A New Dimension in Biological Analysis, R-. H. Kennet et al., eds., Plenum Press, New York and London (1980) .

Serum GP73 levels can be detected by aptamer-based assays, which are very similar to antibody-based assays, but with the use of an aptamer instead of an antibody. Additionally, an aptamer-based assay can be broader, in that nucleic acid amplification (e.g., PCR) and detection assays (e.g., hybridization blots) can also be employed in the detection of GP73 An aptamer can be any polynucleotide, generally a RNA or a DNA, which has a useful biological activity in terns of biochemical activity or molecular recognition attributes. Usually, an aptamer has a molecular activity such as having an enzymatic activity or binding to a polypeptide at a specific region (i.e., similar to an epitope for an antibody) of the polypeptide. It is generally known in the art that an aptamer can be made by in vitro selection methods.

In vitro selection methods include a well known method called systematic evolution of ligands by exponential enrichment (a.k.a. SELEX). Briefly, in vitro selection involves screening a pool of random polynucleotides for a particular polynucleotide that binds to a biomolecule, such as a polypeptide, or has a particular activity that is selectable. Generally, the particular polynucleotide represents a very small fraction of the pool, therefore, a round of amplification, usually via polymerase chain reaction, is employed to increase the representation of potentially useful aptamers. Successive rounds of selection and amplification are employed to exponentially increase the abundance of a particular aptamer. In vitro selection is described in Famulok, M.; Szostak, J. W., In Vitro Selection of Specific Ligand Binding Nucleic Acids, Angew. Chem. 1992,104, 1001. *(*Angew. Chem. Int. Ed. Engl. 1992, 31, 979-988.); Famulok, M.; Szostak, J. W., Selection of Functional RNA and DNA Molecules from Randomized Sequences, Nucleic Acids and Molecular Biology, Vol 7, F. Eckstein, D. M. J. Lilley, Eds., Springer Verlag, Berlin, 1993, pp. 271; Klug, S.; Farmulok, M., All you wanted to know about SELEX; Mol. Biol. Reports 1994, 20, 97-107; and Burgstaller, P.; Famulok, M. Synthetic ribozymes and the first deoxyribozyme; Arsgew. Chem. 1995, 107, 1303-1306 *(*Angew. Chem. Int. Ed Engl. 1995, 34, 1189-1192), US Patent No. 6,287,765, US Patent No. 6,180,348, US Patent No. 6,001,570, US Patent No. 5,861,588, US Patent No. 5,567,588, US Patent No. 5,475,096, and US Patent No. 5,270,163 .

Substantially pure GP73, which can be used as an immunogen for raising polyclonal or monoclonal antibodies, or as a substrate for selecting aptamers, can be prepared, for example, by recombinant DNA methods. For example, the cDNA of SEQ ID NO: 1 can be cloned into an expression vector by techniques within the skill in the art. An expression vector comprising sequences encoding GP73 can then be transfected into an appropriate, for example bacterial host, whereupon GP73 is expressed. The expressed GP73 can then be isolated by any suitable technique.

For example, GP73 protein can be prepared in the form of a bacterially expressed glutathione S-transferase (GST) fusion protein. Such fusion proteins can be prepared using commercially available expression systems, following standard expression protocols, *e.g*., "Expression and Purification of Glutathione-S-Transferase Fusion Proteins", Supplement 10, unit 16.7, in Current Protocols in Molecular Biology (1990) and Smith and Johnson, Gene 67: 34-40 (1988); Frangioni and Neel, Anal. Biochem. 210: 179-187 (1993) .

Briefly, DNA encoding for GP73 (*e.g*., SEQ ID NO: 1) is subcloned into a pGEX2T vector in the correct reading frame and introduced into E. *coli* cells. Transfectants are selected on LB/ampicillin plates after incubation for 12 to 15 hours at 37°C. The selected transfectants are then grown in liquid cultures in growth media containing isopropyl-β-D-thiogalactoside, to induce expression of the GP73 fusion protein. The cells are harvested from the liquid cultures by centrifugation, the bacterial pellet is resuspended and sonicated to lyse the cells.

To isolate the GST-GP73 fusion protein, the lysate is then contacted with glutathione-agarose beads. The beads, which bind the GST-GP73 fusion protein, are collected by centrifugation and the GST-GP73 fusion protein is eluted. The GST agarose beads are removed by treatment of the fusion protein with thrombin cleavage buffer. The released GP73 protein is recovered and used to raise antibodies as described above.

Antibodies against GP73 -can also be raised by immunizing appropriate hosts with immunogenic fragments of the whole GP73 protein, particularly peptides corresponding to the carboxy terminus of the molecule. Fragments of GP73 protein can be obtained by chemical or enzymatic cleavage of isolated GP73 protein. Alternatively, GP73 protein fragments can be obtained by chemical synthesis of small (*e.g*., 7-10 amino acid) subsequences of SEQ ID NO: 2.

Preferably, an anti-GP73 antibody for use in the present methods comprises a detectable label. The detectable label can be directly attached to the primary anti-GP73 antibody. The detectable label can also be indirectly attached to an anti-GP73 antibody by reacting the anti-GP73 antibody with a secondary antibody, *e.g*., a goat anti-rabbit IgG, which bears a detectable label.

The detectable label can comprise, for example, a radionuclide in the case of a radioimmunoassay; a fluorescent moiety in the case of an immunofluorescent assay; a chemiluminescent moiety in the case of a chemiluminescent assay; or an enzyme which cleavers a chromogenic substrate, in the case of an enzyme-linked immunosorbent assay.

The biomolecular interactions assays for detecting serum GP73 levels typically require only very small serum samples, for example in the order of 0.5 microliters. It is understood, however, that any suitable volume of serum sample can be used in the present methods. It is also understood that the serum sample need not be liquid, but can comprise a dried (*e*.*g*., lyophilized) biological sample comprising serum. Any amount of a dried serum sample can be used in the present methods.

Evaluation of subjects with chronic HBV and HCV infections, including those with cirrhosis of the liver, showed that GP73 levels were not elevated to a statistically significant degree as compared to a control. However, GP73 levels in subjects with HCC were significantly elevated. Thus, elevation in GP73 levels in the serum of a subject who is at risk for HCC is an indication that the subject has developed HCC.

The invention therefore provides a method of monitoring a subject at risk for HCC, wherein the levels of GP73 in the subject's serum are evaluated over time. As used herein, a subject "at risk for HCC" includes subjects who have not been formally diagnosed with HCC, but who have a familial history of HCC, have contracted an HBV or HCV infection or have liver damage of any etiology which may progress to HCC.

In the practice of the method, serum samples are obtained from the subject for at least two time points. The level of GP73 in the serum samples are determined and compared to each other. An elevated serum GP73 level in the sample taken at the later time point, relative the sample from the earlier time point, indicates that the subject has developed HCC.

Preferably, a plurality of serum samples are taken from the subject over the course of several months to several years. For example, serum samples can be taken every 3 months from the time a subject is diagnosed with an HBV or HCV infection, for up to 3, up to 5 or up to 10 years over the course of the viral infection. It is understood that serum samples can be taken at lesser or greater intervals for greater or lesser periods of time.

An "elevated level" of GP73 in a subject's serum sample taken at a later time point, as compared to the serum GP73 in a subject's serum sample taken at an earlier time point, means that the amount of GP73 protein per unit volume or unit mass is greater in the later serum sample than in the earlier serum sample. The level of GP73 can be expressed in absolute units of mass of protein per unit volume or unit mass, or as arbitrary units, as discussed above.

Preferably, the level of GP73 protein in the serum sample taken at the later time point is at least 2-fold, for example at least 3-fold, at least 4-fold, at least 5-fold, at least 6-fold, at least 7-fold, at least 8-fold greater than the GP73 level in the serum sample taken at the earlier time point.

Techniques for obtaining serum samples, and for detecting GP73 levels, are as described above.

The practice of the invention is illustrated by the following non-limiting examples.

### Example 1: Detection of GP73 in serum of hepatocellular carcinoma patients

### Serum samples and patient history

Serum samples were collected with the informed consent of participants and in accordance with procedures approved by the Institutional Review Boards of the Fox Chase Cancer Center, Thomas Jefferson University, and the University of Michigan, where applicable. These samples were used in subsequent examples as well. Two groups of patients were included. A group of HBV-infected and control patients was comprised of 38 male subjects of Chinese ethnic background with a minimum age of 35 years, who resided in the United States at the time of sample collection. Because these patients are from a population where HBV is highly endemic, it is likely that all were infected in infancy or early childhood. HBV infection was established based on HBV surface antigen (HBsAg) positivity and on the detection of HBV-DNA in serum. HBV DNA was detected by a "dot blot" method and has a detection limit of sensitivity of approximately 3x10⁵ genome equivalents per ml (Evans et al., 1998, Cancer Epidemiology, Biomarkers, and Prevention, 7:559-565). A group of HCV infected and control patients was drawn from the liver and liver transplant clinics at the University of Michigan Medical Center between September 2001 and May 2002. The diagnosis of HCV infection was established by HCV antibody positivity and the presence of HCV-RNA in serum. Patients with non-viral causes of liver disease and those with multiple disease etiologies were excluded. The presence of chronic hepatitis, cirrhosis, or HCC was established by histological examination of liver biopsy or explant samples. HCC diagnosis was confirmed by ultrasound imaging and biopsy. Liver function tests (ALT or AST) were determined by immunoassay and the upper limit was considered to be 50 IU/ml. Serum was isolated from blood samples immediately after collection, and serum aliquots were stored at -80°C until testing. Blood samples from the HCC subjects were drawn prior to initiation of HCC treatment.

GP73 expression was then evaluated in serum samples consecutively collected from a larger group of HCV-infected individuals. The diagnosis and histological classification was made as above.

### Measuring α-fetoprotein (AFP)

AFP was measured in the serum samples described above with commercially available immunoassays utilizing enhanced chemiluminescence at the University of Michigan Hospital Clinical Diagnostic Laboratory. The upper limit of normal was 8 ng/ml AFP. The demographic and laboratory data were obtained for all patients. HCC patients were staged according to the UNOS TNM staging system.

### Immunoblot analysis and reagents

Equal volumes of patient sera (0.5 or 1.0 µl/lane) were separated by SDS-PAGE on 4-20% polyacrylamide gradient gels. For normalization, some gels also included a lane containing 0.5 µl of serum from a pool of HCV and HBV negative sources (Sigma Inc., St Louis, MO). Following electrophoretic separation, the proteins were transferred to a PVDF membrane by immunoblotting. The membranes were blocked by incubation blocking buffer (1x TBS is 50 mM Tris-HCl, pH 7.6, 150 mM sodium chloride, 5% non-fat dried milk, and 0.1% Tween 20) for 1 hour at room temperature. The blots were incubated overnight with anti-GP73 rabbit polyclonal antibody at a 1:1000 dilution in blocking buffer with gentle rocking at 4°C (Kladney, 2000, Gene, *supra*). The blots were washed 2 times in blocking buffer at room temperature and incubated with horseradish peroxidase conjugated mouse anti-rabbit secondary antibody (1:4000 v/v) at room temperature for 2 hours. The blots were washed 2 times at room temperature in 1 x TBS-T (TBS containing 0.1% Tween 20) and were developed using the ECL Plus chemiluminescent detection system (Amersham Pharmacia Biotech, Arlington Heights, IL).

Total cell protein lysates from liver tissue of HCV infected HCC samples and HCV infected cirrhotic tissue were prepared as previously described (Kladney et al. 2002, Hepatology 35:1431-1440). Protein concentrations were determined by Bradford Assay (Bio-Rad). Twenty-five µg of total cell protein lysates from liver tissue of HCV-infected patients with cirrhosis or HCC were fractionated by SDS-PAGE on 4-20% polyacrylamide gradient gels and subjected to immunoblot analysis with anti-GP73 rabbit polyclonal antibodies as described above. These techniques were used in subsequent examples as well.

### Results

GP73 levels were compared in HCV-infected patients who also had cirrhosis or HCC. The results are shown in Figure 1. Lanes 1 and 2 are HCC tissue lysates. Lanes 3 and 4 are cirrhotic tissue lysates. Lanes 5 and 6 are sera from the two HCV-infected patients with HCC.

GP73 was clearly detectable in the sera of the two HCV infected patients (Fig 1. lanes 5 and 6). Further, higher levels of GP73 were detected in cell lysates derived from liver biopsy material of HCV patients with HCC, compared to HCV patients with cirrhosis (Fig. 1, lanes 1-4).

In addition to a band of approximately 73 kDa (Daltons x 10³) on the Western blot, which represents a GP73 protein, a band of approximately 25 kDa ± 5 kDa, which is arguably a fragment of GP73, was detected in sera from patients with HCC and patients with HCV, but not in sera from normal control individuals.

### Example 2: Detection of GP73 in serum of hepatocellular carcinoma patients

### Methods

Serum samples were obtained, and immunoblot analyses were performed, as described above. Densitometric analyses of the immunoblots were performed to quantify the amounts of GP73 protein in patient sera, relative to the signal present in the Sigma control serum. The signal for the Sigma control serum was set to a value of 1.0.

GP73-specific signals from the 73 kDa species were quantified from X-ray film using an AlphaInnotech FluorChem CCD camera with AlphaEase spot densitometry software, and expressed as integrated intensity units relative to the GP73 signal detected in Sigma control serum (lane S on each blot). Values were calculated as the mean of duplicate or triplicate determinations for each serum sample and results.

Aliquots of human serum (0.5 µl) were obtained from the following HBV study subjects: HBV-negative (Group A); HBV carrier with inactive infection (Group B); HBV carrier with active infection (Group C); HBV-associated HCC (Group D). Aliquots of human serum (0.5 µl) were obtained from the following HCV study subjects: HCV-negative (Group 1); chronic HCV infection (Group 2); HCV-related cirrhosis (Group 3); HCV-related HCC (Group 4). Samples were resolved by SDS-PAGE on 4-20% polyacrylamide gradient gels and subjected to immunoblot analysis with anti-GP73 specific antibody. Commercially obtained, pooled sera from HCV-, HIV-, and HBV-negative subjects (S) were used for normal controls. The demographics and serological profiles of control subjects and patients are summarized in Tables 1 (HBV patients) and 2 (HCV patients), below.

### Results

GP73 levels in the sera of HBV-infected patients with or without HCC were compared to GP73 levels in the sera of HCV-infected patients with or without HCC, and to GP73 levels in control subjects. The results are shown in Figures 2A and 2B. The number assigned to each study subject for identification purposes is indicated above each lane.

High levels of GP73 were present in the sera of 23 of 24 patients with chronic HBV-or HCV-related HCC, but not in healthy individuals without viral hepatitis. Elevated serum levels were also present in a subset of patients with liver cirrhosis without HCC (Figs. 2A and 2B). Low levels of GP73 were detected in the circulation of uninfected individuals, similar to the levels seen in the control serum (S).

Densitometric analyses of the immunoblots of Figs. 2A and 2B were performed as described above to quantify the amounts of GP73 protein in patient sera, relative to the signal present in the Sigma control serum. The results of the densitometric analyses are shown graphically in Figs. 3A and 3B.

The statistical analysis of Fig. 3A shows that the only significant difference is between Group D (HCC patients) versus Groups A, B, and C, p< 0.001. Statistical analysis of HCV patients and controls, as indicated in Fig. 3B, shows that the only significant difference is between Group 4 (HCC patients) versus Groups 1, 2, and 3, p< 0.001.

A few individuals with HBV- or HCV-associated nonmalignant liver disease had somewhat elevated levels of GP73 in sera, while the highest levels were found in patients with a diagnosis of HCC (Fig. 3A and 3B). Seven of the eight individuals with HBV-associated HCC showed an increase greater than any patient in the other three groups (Fig. 2A, Group D and Fig. 3A). Eleven of sixteen individuals with HCV associated HCC showed GP73 levels higher than any patient in the other groups of that experimental set (Fig. 2B, Group 4, and Fig. 3B).

Statistical analyses revealed a statistically significant overall increase in serum GP73 levels in patients with HCC, compared to all other diagnostic groups, p < 0.001 (Fig. 3). Analysis of variance (ANOVA) according to HBV or HCV infection indicated that this difference was present regardless of the viral agent. For each group, the null hypothesis of no difference between the four subject groups was rejected (for the HBV group, F = 49.47, p < 0.0001, for the HCV group, F = 17.51, p < 0.0001). In pairwise tests adjusted for multiple comparisons, the means for each of the groups were compared. In separate analyses of the HBV and HCV datasets, the HCC group differed significantly from each of the other three groups (p < 0.0001). However, the three non-HCC groups were not significantly different from one another. Without wishing to be bound by any particular theory, these results indicate that the appearance of high levels of circulating GP73 may be a feature of virus induced hepatocellular cancer, regardless of the viral etiology of hepatitis (HBV or HCV).

**Table 1. HBV patient demographics and serological profiles of Group A-D subjects**

| **Group** | **Diagnosis** | **serological profile** ¹ | **number and gender of patients ²** | **age (mean ± S.D.)** |
|---|---|---|---|---|
| A | HBV-negative | HBsAg - HBV DNA - normal LFTs | 7 (7M) | 60.1±5.3 |
| B | HBV carrier, inactive | HBsAg + HBV DNA - normal LFTs | 11 (11M) | 52.6±11.4 |
| C | HBV carrier, active | HBsAg + HBV DNA + abnormal LFTs | 12 (12M) | 55.1±9.0 |
| D | HBV-HCC | HBsAg + | 8 (8M) | 57.1±8.8 |

| | | | | |
|---|---|---|---|---|
| ¹LFT = liver function test ²M = Male | | | | |

**Table 2. HCV patient demographics and serological profiles of Group 1-4 subjects**

| **Group** | **Diagnosis** | **serological profile** | **number and gender of patients¹** | **age (mean** ± **S.D.)** |
|---|---|---|---|---|
| 1 | HCV negative | HCV Ab (-), HCV-RNA (-) | 16 (4M,12F) | 41 +/- 13 |
| 2 | HCV chronic | HCV Ab (+), HCV-RNA (+) | 16 (7M,9F) | 45 +/- 6.5 |
| 3 | HCV + cirrhosis | HCV Ab (+), HCV-RNA (+) | 16 (10M,6F) | 49+/-.6.6 |
| 4 | HCV + HCC | HCV Ab (+), HCV-RNA (+) | 16 (14M,2F) | 56+/-12.4 |

| | | | | |
|---|---|---|---|---|
| ¹M = Male, F = Female | | | | |

### Example 3: Comparison of serum GP73 and AFP in detecting HCC

Based on the results obtained in Example 2, a larger blinded study was performed, focusing on a well-characterized HCV-infected cohort (n=142). The levels of GP73 and AFP were measured in sera from patients with HCV-associated liver disease and control patients. Patient groups and demographics are defined in Table 3.

**Table 3. Demographics of the larger cohort of patients with Hepatitis C.**

| **Variable** | **Normal (n=40)** | **Chronic Hepatitis (n=35)** | **Cirrhosis (n=35)** | **HCC (n=33)** | **P value** |
|---|---|---|---|---|---|
| Age | 51±9.7 | 54±6 | 51±8 | 51±10 | 0.14 |
| Gender (M:F) | 30:10 | 20:14 | 16:9 | 28:5 | 0.32 |
| AFP (ng/ml) | 2.94±1.6 | 10.8±23 | 19.7±38 | 11788±60359 | <0.001 |
| %<20 | 100 | 88 | 77 | 55 | |
| % 20-200 | 0 | 12 | 23 | 24 | |
| % >200 | 0 | 0 | 0 | 21 | |
| ALT (IU/ml) | 28.6±9 | 67±41 | 112±124 | 81±49 | <0.001^{#} |
| AST (IU/ml) | 22±5 | 53±36 | 94±85 | 109±59 | 0.003* |
| Bilirubin (mg/dl) | 0.4±0.2 | 0.5±0.4 | 0.9±0.6 | 1.2±0.9 | 0.13 |
| MELD score | 5±0.2 | 6.1±0.4 | 7.8±1.8 | 8.3±2.1 | 0.03* |
| TNM State % (I/II/III/IV) | NA | NA | NA | 9/12/6/6 | |

| | | | | | |
|---|---|---|---|---|---|
| ^{#}Group 3 versus 1 and 2. * Group 4 versus 1 and 2. NA = not applicable. | | | | | |

### Methods

Immunoblots and densitometric analyses were performed as described above.

### Statistical Analysis

Log transformation was used on the AFP values to account for the large range of values. The descriptive statistics for AFP and GP73 were compared by box plots and then by ANOVA. Group differences in means were tested by using SAS V 8.01 (SAS Institute, Cary, NC) PROC GLM for analysis of variance, which uses the least-squares method to fit general linear models, for continuous variables. For binary variables, chi-square was utilized to compare groups. To account for multiple comparisons, p-values for individual means within the ANOVAs were adjusted using the Tukey-Kramer test. To determine the optimal cutoff value for GP73 and AFP in the diagnosis of HCC, Receiver Operating Characteristic (ROC) curves were constructed using all possible cutoffs for each assay. The area under the ROC (AUROC) curves were calculated and compared as described previously (Griner et al., 1981, Ann. Intern. Med. 94:555-600; Metz, 1998, Semin- Nucl. Med. 8:283-298 ). A bivariate normal distribution for the two markers was assumed. A 2-tailed p value of < 0.05 was used to determine statistical significance. All analyses were performed using SAS (Cary, NC, USA).

Immunoblot analysis was used to detect and quantify GP73 levels in sera. For statistical analyses, the subjects were separated into four categories, as before: normal controls (uninfected with HBV or HCV), chronic HCV without cirrhosis, chronic HCV with cirrhosis, and chronic HCV with cirrhosis and HCC. The groups are well-matched for age, gender, and ethnicity (see Table 3).

### Results

The results (Fig. 4A) showed a statistically significant elevation of serum GP73 in patients diagnosed with HCC, compared with nonmalignant liver disease or control groups (ANOVA, p < 0.001). The frequencies of elevation of GP73 (Fig. 4A) were compared with elevations of AFP in patients from this population (Fig. 4B). AFP levels were determined by standard clinical assays as described above. Although AFP levels generally increased in patients with HCC, no statistically significant difference could be demonstrated in AFP levels between the HCC patients and the other groups in this analysis (ANOVA, p = 0.292).

A ROC curve analysis was also performed to compare the sensitivity and specificity of GP73 and AFP in distinguishing patients with cirrhosis from those with cirrhosis plus HCC in this population of individuals chronically infected with HCV. The data derived for Groups 3 and 4 in Figure 4 were plotted for ROC analysis, and are presented in Figure 5. The ROC curves show that GP73 levels are more predictive than AFP as an indicator of HCC. GP73 has a sensitivity of 0.85 and specificity of 0.65, while AFP is lower in both sensitivity (0.70) and specificity (0.60). Based on the assay used, the optimal cut-off value for GP73 was determined to be 8.4 relative units above the GP73 signal from the control sample, and the optimal cut-off for AFP was 9.9 ng/ml. P = 0.149 for the difference between AFP and GP73.

Without wishing to be bound by any particular theory, these studies indicate that serum GP73 levels may be more predictive than AFP for distinguishing between a clinical diagnosis of HCC and nonmalignant liver disease associated with either HBV or HCV infection.
- Therefore, the present invention should not be limited to any single embodiment, but rather should be construed in breadth and scope in accordance with the recitation of the appended claims.

### SEQUENCE LISTING

<110> THOMAS JEFFERSON UNIVERSITY
   SAINT LOUIS UNIVERSITY
   THE UNITED STATES OF AMERICA as represented by the DEPARTMENT OF VETERANS AFFAIRS
   Patrick R. Romano
   Timothy M. Block
   Claus J. Fimmel
   Olga Nikolaeva
<120> DIAGNOSIS AND MONITORING OF
   HEPATOCELLULAR CARCINOMA
<130> 08321-141PC1
<150> 60/500,657
   <151> 2003-09-05
<160> 2
<170> FastSEQ for windows version 4.0
<210> 1
   <211> 3042
   <212> DNA
   <213> Homo sapiens
<400> 1
<210> 2
   <211> 400
   <212> PRT
   <213> Homo sapiens
<400> 2

## Claims

1. A method of diagnosing HCC (hepatocellular carcinoma) in a subject, comprising:
(1) determining the level of GP73 or a polypeptide of 25 kDa +/-5 kDa which cross reacts with a GP73-specific antibody, in a serum sample obtained from a subject suspected of having HCC; and
(2) comparing the level of GP73 or said polypeptide in the serum sample to the serum levels of GP73 or said polypeptide in a control, wherein elevated levels of GP73 or said polypetide in the serum sample relative to the control indicate that the subject has HCC.

2. A method of monitoring a subject at risk for developing HCC (hepatocellular carcinoma), comprising:
determining the levels of GP73 or a polypeptide of 25 kDa +/- 5 kDa which cross reacts with a GP73-specific antibody, in at least a first serum sample obtained from a subject at a first time point and at least a second serum sample obtained from the subject at a second time point, wherein an increase in the level of GP73 or said polypeptide in the second serum sample relative to the level of GP73 or said polypeptide in the first serum sample indicates that the subject has developed HCC.

3. The method of claim 1 or 2, wherein the level of GP73 or said polypeptide in the serum sample relative to the level of GP73 or said polypeptide in the control, or the level of GP73 or said polypeptide in the second serum sample relative to the level in the first serum sample, is elevated at least 2-fold.

4. The method of claim 1 or 2, wherein the level of GP73 or said polypeptide in the serum sample relative to the level of GP73 or said polypeptide in the control, or the level of GP73 or said polypeptide in the second serum sample relative to the level in the first serum sample, is elevated at least 3-fold.

5. The method of claim 1 or 2, wherein the level of GP73 or said polypeptide in the serum sample relative to the level of GP73 or said polypeptide in the control, or the level of GP73 or said polypeptide in the second serum sample relative to the level in the first serum sample, is elevated at least 6-fold.

6. The method of any preceding claim, wherein the level of GP73 or said polypeptide in a serum sample is detected by a biomolecular interaction assay.

7. The method of claim 6, wherein the biomolecular interaction assay is an immunoassay.

8. The method of claim 7, wherein the immunoassays is selected from the group consisting of Western blot analysis, radioimmunoassay (RIA), immunofluorescent assay, chemiluminescent assay, or enzyme-linked immunosorbent assay (ELISA).

9. The method of any one of claims 1 to 4, wherein the level of GP73 or said polypeptide in a serum sample is detected by an aptamer.

10. The method of any one of claims 1 to 4, wherein the level of GP73 or said polypeptide in a serum sample is detected by an antibody.

11. The method of claim 10, wherein the antibody comprises a polyclonal or a monoclonal anti-GP73 antibody.

12. The method of claim 10, wherein the antibody comprises an antibody fragment.

## Patentansprüche

1. Ein Verfahren zum Diagnostizieren eines HCC (hepatozellulären Karzinoms) bei einem Patienten, das Folgendes beinhaltet:
(1) Bestimmen des Gehalts von GP73 oder eines Polypeptids von 25 kDa +/- 5 kDa, das mit einem GP73-spezifischen Antikörper kreuzreagiert, in einer Serumprobe, die einem Patienten, welcher im Verdacht steht, HCC zu haben, entnommen wurde; und
(2) Vergleichen des Gehalts von GP73 oder des Polypeptids in der Serumprobe mit dem Serumgehalt von GP73 oder des Polypeptids in einer Kontrolle, wobei ein erhöhter Gehalt von GP73 oder des Polypeptids in der Serumprobe relativ zu der Kontrolle anzeigt, dass der Patient HCC hat.

2. Ein Verfahren zum Überwachen eines Patienten, welcher gefährdet ist, HCC (hepatozelluläres Karzinom) zu entwickeln, das Folgendes beinhaltet:
Bestimmen des Gehalts von GP73 oder eines Polypeptids von 25 kDa +/- 5 kDa, das mit einem GP73-spezifischen Antikörper kreuzreagiert, in mindestens einer ersten Serumprobe, die einem Patienten zu einem ersten Zeitpunkt entnommen wurde, und mindestens einer zweiten Serumprobe, die dem Patienten zu einem zweiten Zeitpunkt entnommen wurde, wobei eine Erhöhung des Gehalts von GP73 oder des Polypeptids in der zweiten Serumprobe relativ zu dem Gehalt von GP73 oder des Polypeptids in der ersten Serumprobe anzeigt, das der Patient HCC entwickelt hat.

3. Verfahren gemäß Anspruch 1 oder 2, wobei der Gehalt von GP73 oder des Polypeptids in der Serumprobe relativ zu dem Gehalt von GP73 oder des Polypeptids in der Kontrolle oder der Gehalt von GP73 oder des Polypeptids in der zweiten Serumprobe relativ zu dem Gehalt in der ersten Serumprobe mindestens doppelt so hoch ist.

4. Verfahren gemäß Anspruch 1 oder 2, wobei der Gehalt von GP73 oder des Polypeptids in der Serumprobe relativ zu dem Gehalt von GP73 oder des Polypeptids in der Kontrolle oder der Gehalt von GP73 oder des Polypeptids in der zweiten Serumprobe relativ zu dem Gehalt in der ersten Serumprobe mindestens dreimal so hoch ist.

5. Verfahren gemäß Anspruch 1 oder 2, wobei der Gehalt von GP73 oder des Polypeptids in der Serumprobe relativ zu dem Gehalt von GP73 oder des Polypeptids in der Kontrolle oder der Gehalt von GP73 oder des Polypeptids in der zweiten Serumprobe relativ zu dem Gehalt in der ersten Serumprobe mindestens sechsmal so hoch ist.

6. Verfahren gemäß einem der vorhergehenden Ansprüche, wobei der Gehalt von GP73 oder des Polypeptids in einer Serumprobe durch einen Assay biomolekularer Interaktion entdeckt wird.

7. Verfahren gemäß Anspruch 6, wobei der Assay biomolekularer Interaktion ein Immunoassay ist.

8. Verfahren gemäß Anspruch 7, wobei der Immunoassay aus der Gruppe, die aus Western-Blot-Analyse, Radioimmunoassay (RIA), lmmunofluoreszenzassay, Chemilumineszenzassay oder enzymgekoppeltem Immunadsorptionsassay (ELISA) besteht, ausgewählt ist.

9. Verfahren gemäß einem der Ansprüche 1 bis 4, wobei der Gehalt von GP73 oder des Polypeptids in einer Serumprobe durch ein Aptamer entdeckt wird.

10. Verfahren gemäß einem der Ansprüche 1 bis 4, wobei der Gehalt von GP73 oder des Polypeptids in einer Serumprobe durch einen Antikörper entdeckt wird.

11. Verfahren gemäß Anspruch 10, wobei der Antikörper einen polyklonalen oder einen monoklonalen Anti-GP73-Antikörper beinhaltet.

12. Verfahren gemäß Anspruch 10, wobei der Antikörper ein Antikörperfragment beinhaltet.

## Revendications

1. Une méthode pour diagnostiquer un HCC (carcinome hépatocellulaire) chez un sujet, comprenant :
(1) déterminer le niveau de GP73 ou d'un polypeptide de 25 kDa +/- 5 kDa qui réagit de façon croisée avec un anticorps spécifique à GP73, dans un échantillon de sérum obtenu chez un sujet soupçonné d'avoir un HCC ; et
(2) comparer le niveau de GP73 ou dudit polypeptide dans l'échantillon de sérum aux niveaux de sérum de GP73 ou dudit polypeptide dans un témoin, où des niveaux plus élevés de GP73 ou dudit polypeptide dans l'échantillon de sérum relativement au témoin indiquent que le sujet a un HCC.

2. Une méthode pour surveiller un sujet chez lequel un HCC (carcinome hépatocellulaire) risque de se développer, comprenant :
déterminer les niveaux de GP73 ou d'un polypeptide de 25 kDa +/- 5 kDa qui réagit de façon croisée avec un anticorps spécifique de GP73, dans au moins un premier échantillon de sérum obtenu chez un sujet à un premier point dans le temps et au moins un deuxième échantillon de sérum obtenu chez le sujet à un deuxième point dans le temps, où une augmentation du niveau de GP73 ou dudit polypeptide dans le deuxième échantillon de sérum relativement au niveau de GP73 ou dudit polypeptide dans le premier échantillon de sérum indique qu'un HCC s'est développé chez le sujet.

3. La méthode de la revendication 1 ou de la revendication 2, dans laquelle le niveau de GP73 ou dudit polypeptide dans l'échantillon de sérum relativement au niveau de GP73 ou dudit polypeptide dans le témoin, ou le niveau de GP73 ou dudit polypeptide dans le deuxième échantillon de sérum relativement au niveau dans le premier échantillon de sérum, est au moins 2 fois plus élevé.

4. La méthode de la revendication 1 ou de la revendication 2, dans laquelle le niveau de GP73 ou dudit polypeptide dans l'échantillon de sérum relativement au niveau de GP73 ou dudit polypeptide dans le témoin, ou le niveau de GP73 ou dudit polypeptide dans le deuxième échantillon de sérum relativement au niveau dans le premier échantillon de sérum, est au moins 3 fois plus élevé.

5. La méthode de la revendication 1 ou de la revendication 2, dans laquelle le niveau de GP73 ou dudit polypeptide dans l'échantillon de sérum relativement au niveau de GP73 ou dudit polypeptide dans le témoin, ou le niveau de GP73 ou dudit polypeptide dans le deuxième échantillon de sérum relativement au niveau dans le premier échantillon de sérum, est au moins 6 fois plus élevé.

6. La méthode de n'importe quelle revendication précédente, dans laquelle le niveau de GP73 ou dudit polypeptide dans un échantillon de sérum est détecté par une analyse des interactions biomoléculaires.

7. La méthode de la revendication 6, dans laquelle l'analyse des interactions biomoléculaires est un immunoessai.

8. La méthode de la revendication 7, dans laquelle l'immunoessai est sélectionné dans le groupe consistant en transfert de Western, dosage radioimmunologique (RIA), dosage immunofluorescent, dosage par chimioluminescence, ou dosage immunoenzymatique (ELISA).

9. La méthode de n'importe laquelle des revendications 1 à 4, dans laquelle le niveau de GP73 ou dudit polypeptide dans un échantillon de sérum est détecté par un aptamère.

10. La méthode de n'importe laquelle des revendications 1 à 4, dans laquelle le niveau de GP73 ou dudit polypeptide dans un échantillon de sérum est détecté par un anticorps.

11. La méthode de la revendication 10, dans laquelle l'anticorps comprend un anticorps anti-GP73 polyclonal ou monoclonal.

12. La méthode de la revendication 10, dans laquelle l'anticorps comprend un fragment d'anticorps.
